# EUROPEAN PATENT SPECIFICATION

(11) **EP 4 120 967 B1**
(45) Date of publication and mention of the grant of the patent: **23.09.2026**
(21) Application number: 21772646.2
(22) Date of filing: 16.03.2021
(51) Int. Cl.: A61F 5/01, A61F 5/02

(54) **EXOSUIT HAVING ADJUSTABLE MODULAR STRAPS AND BANDS**
EXOSKELETT MIT VERSTELLBAREN MODULAREN RIEMEN UND BÄNDERN
EXOSQUELETTE MOTORISÉ AYANT DES SANGLES AJUSTABLES ET DES BANDES MODULAIRES

(30) Priority: 16.03.2020 US 202062990312 P
(43) Date of publication of application: 25.01.2023
(73) Proprietor: Vanderbilt University, Nashville TN 37240 (US); Herowear LLC, Nashville, TN 37204 (US)
(72) Inventor: ZELIK, Karl, Nashville, Tennessee 37240 (US); YANDELL, Matthew, Nashville, Tennessee 37240 (US); MARINO, Matthew, Nashville, Tennessee 37240 (US); HARRIS, Mark, Nashville, Tennessee 37240 (US); PAILES-FRIEDMAN, Rebeccah, Nashville, Tennessee 37240 (US); TOMITA, Ryu, Nashville, Tennessee 37240 (US); REAMEY, Paul, Nashville, Tennessee 37240 (US); HUGGINS, Evan, Nashville, Tennessee 37240 (US)
(74) Representative: Loyer & Abello
(86) International application number: PCT/US2021/022531
(87) International publication number: WO 2021/188516

(56) References cited:
- WO-A1-2019/161232
- DE-A1- 102004 009 315
- DE-U1- 202005 011 650
- US-A- 5 256 135
- US-A1- 2005 130 815
- US-A1- 2005 130 815
- US-A1- 2010 125 230
- US-A1- 2014 100 501
- US-A1- 2017 209 330
- US-A1- 2018 193 686
- US-A1- 2019 358 074
- US-B1- 6 190 342
- US-B1- 6 450 131

## Description

### CROSS REFERENCE TO RELATED APPLICATION(S)

### GOVERNMENT SPONSORSHIP

This invention was made with government support under SBIR grant 1913763 awarded by the National Science Foundation and SBIR grant R43OH011872 awarded by the Centers for Disease Control and Prevention. The government has certain rights in the invention.

### FIELD OF THE INVENTION

The present invention relates to wearable assistance devices such as exosuits/exoskeletons, and more specifically to wearable assistance devices for providing assistive force while reducing lower back muscle stress, fatigue, injury and/or pain, and even more specifically to upgrades, improvements and extensions to back-assist exosuits.

### BACKGROUND OF THE INVENTION

Lower back pain is a disabling condition experienced by a high percentage of adults within their lifetimes. It is the leading cause of limited physical activity and the second leading cause of missed work in the U.S. and a significant economic burden. Lower back pain is estimated to cost $130-230 billion per year in the U.S. due to medical expenses and lost worker productivity.

Lower back pain is particularly common among individuals who perform repetitive or heavy lifting, due to elevated loading on the lumbar spine and muscles that predisposes them to injury risk. Elevated and even moderate loads, applied repetitively to the lumbar spine and muscles can increase the risk of lower back pain, weaken or damage the vertebral bodies, strain muscles, and cause intervertebral disc degeneration and herniation. Prolonged leaning and other static postures are also potential risk factors for lower back pain. Combined compression and bending applied repetitively to cadaveric human lumbar spines often causes intervertebral disc damage. Similarly, elevated and repetitive loading of tissues such as muscles and ligaments can cause strains and damage.

The loading of lumbar muscles, ligaments, vertebrae and discs occurs repeatedly throughout the day during activities such as bending, lifting, and even sitting. The majority of loading on the lumbar spine is the result of back muscles. Back muscles produce large forces and act at short moment arms about the intervertebral joints to balance moments from the upper-body and external objects. The lumbar spine experiences a large flexion moment during forward leaning of the trunk due to the weight of the upper-body and any additional external loads. To keep the upper-body from falling forward, the flexion moment must be counter-balanced by an extension moment. The extension moment is provided by posterior lumbar muscles which apply forces roughly parallel to the spine. This compressive force caused by the back extensor muscles is exerted on the spine and can cause damage and pain.

Assistance devices such as wearable robots have been designed for specific industrial work tasks or environments, but have form-factors that render them too bulky and impractical for daily at-home use or use in other business, social, clinical, and industrial settings. For example, to maximize the moment arm and thus mechanical advantage, some assistance devices are designed with components that protrude significantly from the lower back. For a daily user, these design features can be restrictive, inhibiting basic activities such as sitting, lying down, stair ascent/descent, or navigating typical home or work environments. Moreover, many of these devices lack the adjustability to fit different body shapes and sizes. Unisex designs are common in the exoskeleton and wearable robot industry, and often, devices fit the male body better than the female body, which results is discomfort and device disuse. Another common problem is that a certain component or aspect of an exoskeleton works fine for one application but is impractical for another, rendering the entire device impractical for the latter use case.

Commercially-available back belts and braces also have not reduced back pain or injury. Often these belts and braces operate by restricting motion of the spine, and attempt to increase intra-abdominal pressure to reduce forces on the spine.

US 6 190 342 B1 discloses an harness for the alleviation of back strain has members passing over the shoulders and around the thoraric region with attachments to a pivotable tension equalizing plate whereby the lumbar or lower back region is supported and a balance between the anterior and posterior muscle groups is attained.

The device disclosed by DE 10 2004 009315 A1 has a frame assembled of an upper and a lower part. The upper part is provided with two hooks to be hung over the shoulders of the user. The lower part is u-shaped and bent away from the body in the lower area. Both parts are joined with screws and can be adjusted as required. A vertical strap divided into an upper elastic and a lower flexible segment is attached to the upper frame part and guided across the lower segment of the lower part. Two lateral straps, exceeding from the central strap, can be guided around the thighs.

What is needed is an assistance device that can reduce strain on back muscles and spinal discs during bending and lifting without restricting spine motion. Further, there is a need for such an assistance device to be lightweight, unobtrusive and simple to put on and take off. Finally, it is critical for the device to fit each user properly and comfortably, particularly if it will be worn for prolonged periods of time. Ideally, key components of the assistance device would also be easily personalizable or customizable.

Thus, it is desirable to provide a wearable assistance device and method of using same that are able to overcome the above disadvantages, and that meet the needs of end-users. In order to address the above problems, several improvements, upgrades, and/or extensions have been developed that may be used to contribute, either singularly or in combination, to enhanced form, fit and/or function of an exosuit, and ensure the exosuit can be personalized for users or use cases as needed. Each of these several concepts are described below.

Advantages of the present invention will become more fully apparent from the detailed description of the invention hereinbelow.

### SUMMARY OF THE INVENTION

The invention is defined by independent claim 1. Advantageous embodiments are defined by the dependent claims. Embodiments are directed to a wearable assistance device that includes an upper-body interface, a plurality of fasteners and a back component. The back component is configured to detachably connect to the upper-body interface which is selectable from amongst multiple upper-body interfaces comprising trunk straps of varying shapes or lengths to accommodate users with different shapes or statures. The upper-body interface is configured to be detachably connected to the back component via the plurality of fasteners.

Embodiments are also directed to a wearable assistance device that includes an upper-body interface, a lower-body interface, and a modular elastic band. The modular elastic band is detachably connected between the upper-body interface and the lower-body interface.

Embodiments are further directed to a wearable assistance device that includes an upper-body interface, a lower-body interface, a plurality of fasteners, and a back component. The back component is configured to detachably connect to the upper-body interface which is selectable from amongst multiple upper-body interfaces comprising trunk straps of varying shapes or lengths to accommodate users with different shapes or statures. The upper-body interface is configured to be detachably connected to the back component via the plurality of fasteners.

Additional embodiments and additional features of embodiments for the wearable assistance device are described below and are hereby incorporated into this section.

### BRIEF DESCRIPTION OF THE DRAWINGS

The foregoing summary, as well as the following detailed description, will be better understood when read in conjunction with the appended drawings. For the purpose of illustration only, there is shown in the drawings certain embodiments. It is understood, however, that the inventive concepts disclosed herein are not limited to the precise arrangements and instrumentalities shown in the figures. The detailed description will refer to the following drawings in which like numerals, where present, refer to like items.
**FIG. 1A** is a drawing illustrating a rearward view of male-fit straps for an exosuit without a back component;
**FIG. 1B** is a drawing illustrating a rearward view of female-fit straps for an exosuit without a back component;
**FIG. 2A** is a drawing illustrating a rearward view of a back component for an exosuit;
**FIG. 2B** is a drawing illustrating a rearward, perspective view of the back component shown in **FIG. 2A****,** with a top end of the back component pulled open;
**FIG. 3** is a drawing illustrating a rearward view of straps detachably connected to a back component of an exosuit;
**FIG. 4A** is a drawing illustrating a frontward view of a male on-body rendering of an exosuit with a back component;
**FIG. 4B** is a drawing illustrating a rearward view of the male on-body rendered exosuit shown in **FIG. 4A****;**
**FIG. 5** is a drawing illustrating a rearward view of modular elastic bands for an exosuit with a back component;
**FIG. 6A** is a drawing illustrating an exploded, perspective schematic view of a switch system for an exosuit;
**FIG. 6B** is a drawing illustrating a non-exploded, perspective view of the switch system shown in **FIG. 6A** in disengaged mode and including a connected Bowden cable;
**FIG. 7** is a drawing illustrating an exploded, elevated schematic view of a clutch mechanism for an exosuit;
**FIG. 8** is a drawing illustrating a rearward view of a back component and detached clutch mechanism for an exosuit, including locking pins/studs (embedded within or attached to the back component) and complementary locking holes on the rear side of the clutch mechanism;
**FIG. 9A** is a drawing illustrating a rearward view of a male on-body rendering of an exosuit with detachable thigh sleeve anchor leads attached to a waist belt, and also a detachable linkage between the back component and the waist belt;
**FIG. 9B** is a drawing illustrating a side view of the exosuit shown in **FIG. 9A****,** depicting the user in a standing position; and
**FIG. 9C** is a drawing illustrating a side view of the exosuit shown in **FIG. 9A****,** depicting the user in a forward-leaning position.

### DETAILED DESCRIPTION OF THE INVENTION

It is to be understood that the figures and descriptions of the present invention may have been simplified to illustrate elements that are relevant for a clear understanding of the present invention, while eliminating, for purposes of clarity, other elements found in a typical exosuit or wearable assistance device. Those of ordinary skill in the art will recognize that other elements may be desirable and/or required in order to implement the present invention. However, because such elements are well known in the art, and because they do not facilitate a better understanding of the present invention, a discussion of such elements is not provided herein. It is also to be understood that the drawings included herewith only provide diagrammatic representations of the presently preferred structures of the present invention and that structures falling within the scope of the present invention may include structures different than those shown in the drawings. Reference will now be made to the drawings wherein like structures are provided with like reference designations.

Before explaining at least one embodiment in detail, it should be understood that the inventive concepts set forth herein are not limited in their application to the construction details or component arrangements set forth in the following description or illustrated in the drawings. It should also be understood that the phraseology and terminology employed herein are merely for descriptive purposes and should not be considered limiting.

It should further be understood that any one of the described features may be used separately or in combination with other features. Other invented devices, systems, methods, features, and advantages will be or become apparent to one with skill in the art upon examining the drawings and the detailed description herein. It is intended that all such additional devices, systems, methods, features, and advantages be protected by the accompanying claims.

For purposes of this disclosure, the phrase "body segment" may include a body part such as a back, lumbar spine, hip, neck, etc., or a body joint such as an ankle, knee, elbow, wrist, etc., and thus, may all be used interchangeably. Also, the phrase "body segment" may include multiple body parts or body joints.

For purposes of this disclosure, the phrase "mechanical advantage" may include "assistive force", "assistive torque", or "assistive moment of force", and thus, may all be used interchangeably.

For purposes of this disclosure, the phrase "wearable assistance device" may be an exosuit, exoskeleton, or other device that provides mechanical advantage about a body segment of a user.

For purposes of this disclosure, the phrases "elastic band" and "elastic member" may be used interchangeably, and may be any member that has an amount of elasticity associated with it and which can take the form of, for example, a spring, cable, string, strap, cord, webbing, rope, band, beam, gas-spring, pneumatic, etc., and may be coiled or non-coiled.

For purposes of this disclosure, the phrases "upper body interface" and "lower body interface" refer to body interfaces that can be positioned anywhere on the user's body, with the upper body interface placed higher relative to the lower body interface, assuming the user is in a standing/vertical position.

For purposes of this disclosure, the phrase "clutch mechanism" may be any member that provides the user of an exosuit with the ability to adjust the assistive force level applied by an elastic band, and which can take the form of, for example, a rotational pawl and ratchet mechanism (comprising, for example, a coil/rotor spring), friction-based clutch, cam clutch, overrunning clutch, plate clutch, electromagnetic clutch, positive clutch, buckle, latch, freewheel clutch, unidirectional clutch, etc., and may be powered or non-powered. Powered embodiments may include, for instance, a motor that engages/disengages the clutch, or a powered actuator (e.g., motor with or without battery and motor controller electronics) acting in combination with a clutch as part of its power transmission system. The clutch mechanism may comprise the motor and/or powered actuator. Although, in general, powered actuators and clutches are not the same, the powered actuator may itself be considered a type of clutch mechanism for purposes of this disclosure relating to modular, swappable or detachable components.

### Modular Back/Straps

This feature provides a unique way to create trunk attachments of exosuits and exoskeletons which enables the ability to have cost-effective and gender-specific straps (for males vs. females, or for other body types or customized applications). This helps minimize the number of sizes and SKUs of a device which provides advantages and efficiencies with respect to manufacturing, inventory and commercialization, and being able to personalize and perfect device fit for a broad range of users (men and women of different shapes and sizes).

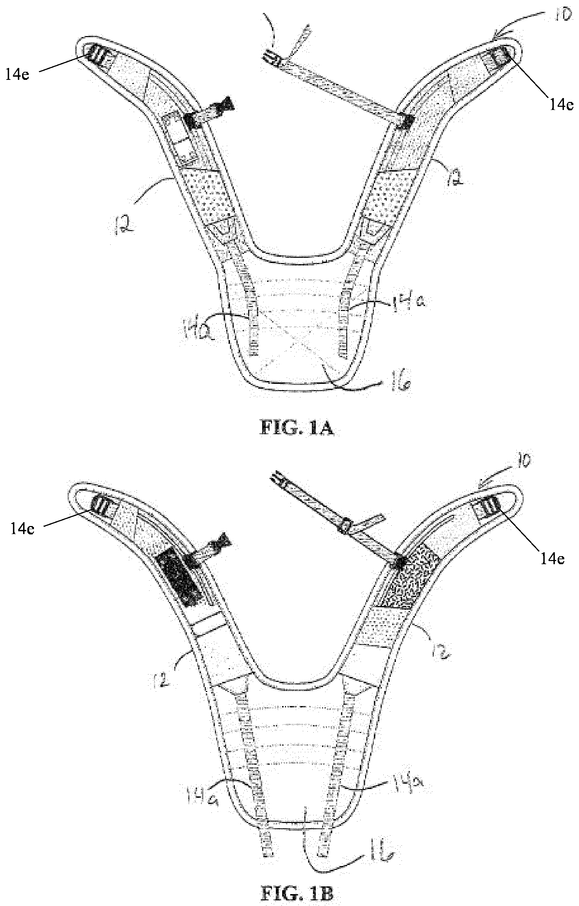

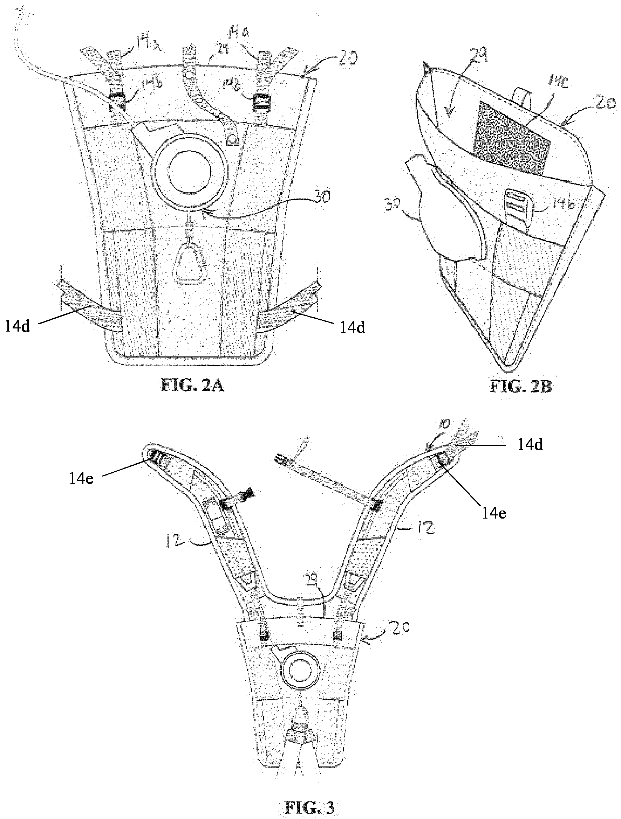

**FIG. 1A** is a drawing illustrating a rearward view of male-fit trunk straps 12 (which are part of an upper-body interface 10) for an exosuit without a back component (*see* back component 20 in **FIGS. 2A-3** below). In an embodiment, and as depicted in **FIG. 1A****,** the straps 12 can be made in two or more different versions, for example: female-fit (which is designed with a specific curvature that better fits around the female body and breasts) and male-fit, each of which has different shapes to best fit the bodies of females and males, or could be customized for specific body types. **FIG. 1B** is a drawing illustrating a rearward view of female-fit straps for an exosuit without a back component. In other examples, strap versions could vary based on users' different shapes or statures. In any of the above scenarios, the straps 12 are detachably connected to the back component 20 via fasteners in the form of, for example, webbing 14a with buckles 14b, as shown in **FIG. 2A****.** The buckles are shown as being connected to (provided on) the back component 20, but may alternatively be connected to (provided on) the straps 12.

**FIG. 2A** is a drawing illustrating a rearward view of the back component 20 for an exosuit. In an embodiment, and as depicted in **FIG. 2A****,** the back component 20 holds a clutch mechanism 30, and has a pocket 29 (*see* **FIG. 2B****)** in it with an opening along the top (or another type of connector or attachment mechanism for housing/affixing the webbing 14a and/or bottom flap 16 of the straps 12). The buckle 14b is attached to the back component 20 and may reside on the rear exterior surface of the back component 20 (as best seen in **FIG. 2B****),** or the buckle 14b may be located within the pocket 29. **FIG. 2B** is a drawing illustrating a rearward, perspective view of the back component 20 shown in **FIG. 2A****,** with a top end of the back component 20 pulled open depicting the pocket 29.

In this embodiment, the modularity of the exosuit is achieved because the bottom flap 16 of the straps 12 can slip inside the pocket 29 of the back component 20 (and may be affixed via, for example, hook and loop fastener 14c within the pocket 29). This means one can easily swap male and female straps 12 onto the same back component 20. There is also adjustability, so that the straps 12 or webbing 14a can be slipped in deeper into the pocket 29 for people with shorter torsos, or can be set higher for people with longer torsos. Thus, the modularity design also provides height adjustability to fit people of different statures. **FIG. 3** is a drawing illustrating a rearward view of the straps 12 detachably connected to a back component 20 of an exosuit. This figure shows the bottom flap 16 of the straps 12 inserted into the pocket 29 of the back component 20.

More specifically, in an embodiment, the straps 12 and back component 20 are held together by a combination of hook and loop fastener pad 14c which is placed inside the pocket 29, when connected with a corresponding hook and loop end portion of the bottom flap 16 of the upper-body interface 10. The buckles 14b detachably securing the webbing 14a to the back component 20 may additionally or alternatively be employed. But other detachable connectors could alternatively be used in place of the hook and loop fasteners and/or buckles 14b. In another embodiment, the straps 12 may be integral with the back component 20, thereby eliminating the need for the above elements such as webbing 14a, buckles 14b, hook and loop fastener pad 14c, bottom flap 16 and pocket 29.

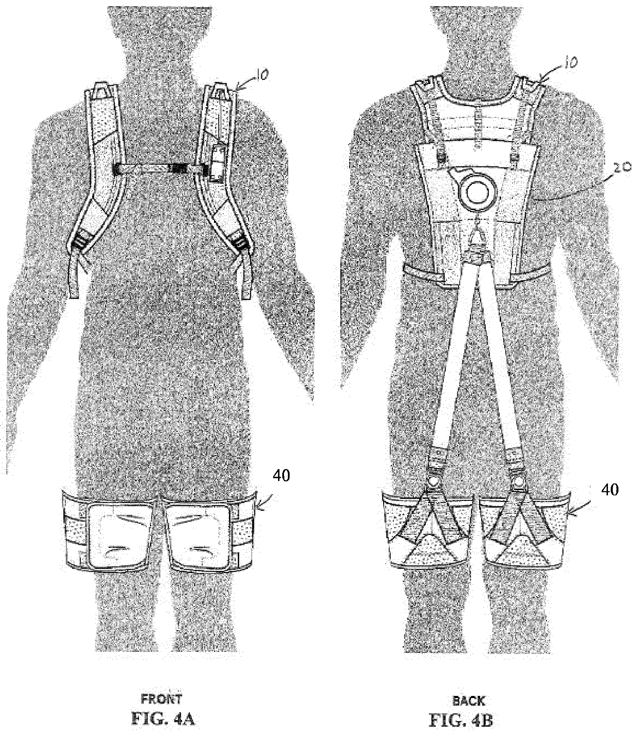

**FIG. 4A** is a drawing illustrating a frontward view of a male on-body rendering of an exosuit with a back component 20. The exosuit includes an upper-body interface 10, back component 20 *(see* **FIG. 4B****),** and lower-body interface 30. **FIG. 4B** is a drawing illustrating a rearward view of the male on-body rendered exosuit shown in **FIG. 4A**

### Modular Elastic Bands

Modularity of elastic bands (i.e., quick swappability of elastic bands) provides a cost-effective way to minimize product SKUs, and also empowers users to select their own preferred level of assistance, or swap in different springs having different properties (e.g., strength, material, stiffness, length, etc.) when needed.

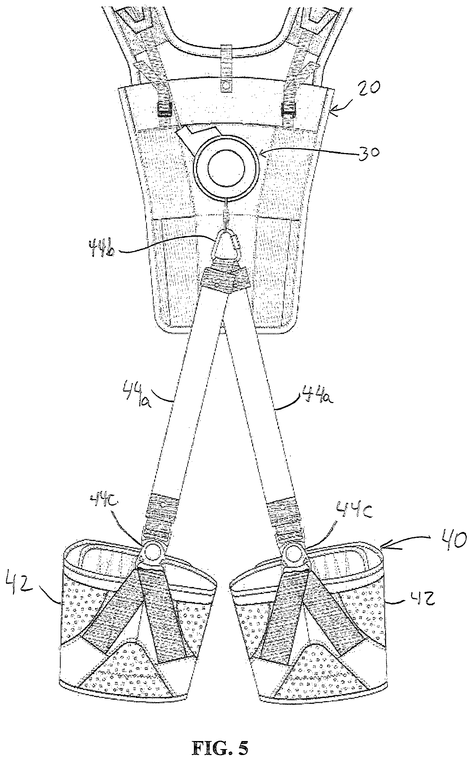

**FIG. 5** is a drawing illustrating a rearward view of modular elastic bands 44a for an exosuit with a back component 20. In an embodiment, and as depicted in **FIG. 5****,** there are detachable connectors 44c (e.g., quick-release buckles) at the top of each leg or thigh sleeve 42 (or at the top of pants or shorts (*not shown*)) which are part of a lower-body interface 40, and also another connector 44b such as a carabiner (e.g., of triangular type) below the clutch mechanism 30 on the back component 20. The elastic bands 44a connect into these buckles (connectors 44c - at bottom) and carabiner (connectors 44b - at top). Other detachable connectors could be used for this purpose in place of the buckles and/or carabiner. As one example, a pulley could be used in place of the carabiner. Furthermore, a clutch mechanism could be built into the pulley, or alternatively connected above or below the pulley. The detachable connectors 44b and 44c allow the elastic bands 44a to be quickly swapped for different lengths, materials, and/or stiffnesses. That also allows users to select the elastic band length, material, and stiffness that works/feels best for them. Other elastic devices, materials or mechanisms may be substituted for the elastic bands such as springs, or other viscoelastic devices, materials or mechanisms such as dampers could also be used, or other quasi-passive or powered (motorized) elements could also be used here to provide adjustable levels of assistance. The elastic bands 44a may be detachably connected anywhere between an upper-body interface 10 and a lower-body interface 40. For example, one or both ends of the elastic bands 44a may be detachably connected to other element(s) (e.g., a clutch, motor, powered actuator) connected between the upper-body interface 10 and the lower-body interface 40. Or, the elastic bands 44a may be directly detachably connected to the upper-body interface 10 and the lower-body interface 40 with no other element(s) therebetween. As one specific example, the elastic bands 44a may be connected to the shoulder straps 12 without the use of a back component 20 and/or clutch mechanism 30. The connection of the elastic bands 44a to components may occur at a fixed point, or alternatively, may be a sliding contact connection.

In one embodiment the modular elastic bands 44a are partially or fully retractable up into the back component 20 when the elastic bands are detached/disconnected from the thigh sleeves 42 at connectors 44c. In one example, this could be achieved using a coil spring located in the clutch mechanism 30 on the back component 20 that is tensioned such that when the elastic bands 44a are detached from the thigh sleeves 42 the elastic bands 44a are pulled up to a position inside the pouch 29 or on top of or otherwise adjacent to the back component 20. Or in another exemplary embodiment, the elastic bands 44a could be made from a circular elastic cord (rather than a flat elastic band) which could be pulled into the housing that contains the coil spring and clutch mechanism 30. As such, the elastic bands 44a and clutch mechanism 30 could be stored inside an upper-body interface consisting of the back component 20 and straps 12, and then the thigh sleeves 42 could be worn or built directly into pants or shorts. When assistance was needed by the user, the elastic bands 44a could then be pulled down and connected to the thigh sleeves 42 at connectors 44c. And then re-stored in the back component 20 when done, if the user were not planning to use assistance for a period of time. This could have applications for industries or the military where infrequent or intermittent assistance was needed. If the clutch mechanism 30 is located on a component of the exosuit other than the back component 20, then the elastic bands 44a could similarly be configured to partially or fully retract into the clutch mechanism 30 or whichever component to which the clutch mechanism 30 is affixed.

By making the elastic bands modular it also allows the lower-body interface (e.g., thigh sleeves) to be modular. This makes it easier to fit personalized components to each user (e.g., two individuals may be the same height but have very different thigh circumference). Modular thigh sleeves allow proper fitting sleeves for each potentially unique user. It also allows for users to quickly swap in a different thigh sleeve, or a different style of thigh sleeve, enabling additional customization for users and use cases.

### Modular Switch System

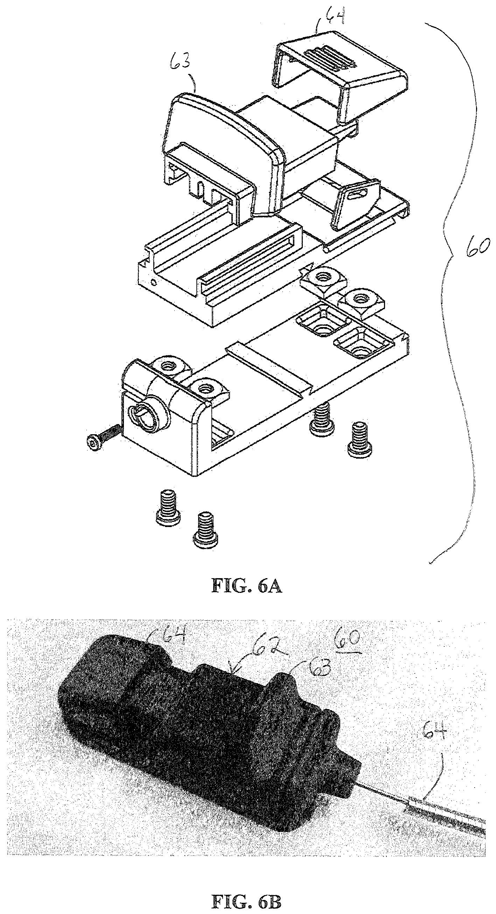

**FIG. 6A** is a drawing illustrating an exploded, perspective schematic view of a switch system 60 for an exosuit. In an embodiment, and as depicted in **FIG. 6A****,** a switch system 60 provides a unique switch mechanism to actuate the clutch mechanism 30. In an embodiment, the switch system 60 uses a "living hinge design" (which uses a thin flexible material such as plastic rather than a traditional rotational joint to provide motion or rotation between two adjacent components). The switch system 60 comprises a main body 62. A notable feature is that the action/motion to engage the switch system 60 (i.e., pulling linearly (and vertically downward) on a slider 63 along a longitudinal axis of the main body 62) is different from the action/motion to disengage the switch system 60 (i.e., pushing horizontally on the button cap 64 towards a user's chest). That provides a very clear distinction to help a user know when the wearable assistance device is being turned on vs. off. Note that the switch system 60 depicted in the figure would preferably be mounted substantially vertically along someone's chest (near the user's shoulder) so that the button cap 64 was at the bottom. Alternatively, the switch system 60 could be placed or mounted elsewhere on the exosuit or user's body. The switch system 60 may also be built directly into or onto the clutch mechanism 30, for instance, in a configuration in which the clutch mechanism 30 is located on the straps 12. When the switch system 60 is built directly into or onto the clutch mechanism 30, the Bowden cable may be eliminated.

The switch system 60 may be detachably connected to the straps 12 via a coupler such as bolt or other quick swap mechanism (e.g., hot shoe, magnetic attachments, locking pins with complementary locking holes). In one embodiment, the switch system 60 is modular and quick swappable, so that a different style of switch could be quickly inserted. Different styles of switches may be used in different industries. For instance, an industry in which workers wear gloves may want a larger switch to make it easier to toggle assistance on/off, whereas another industry may prefer the switch to be smaller or a different style (e.g., knob, dial, slider). Yet another example is that some industries or use cases may require a powered switch, which contains a small battery, electronics, and motor or other actuator (e.g., solenoid) that can be controlled via input from a phone app, or an accelerometer or other sensor or trigger by the user, or from a computer algorithm that uses sensors on the exosuit or in the surrounding environment to trigger assistance on/off. The modularity and swappability of switches is also advantageous for building a versatile and customizable exosuit system that is easy to maintain or repair, and which allows end-users to personalize their device.

**FIG. 6B** is a drawing illustrating a non-exploded, perspective view of the switch system 60 shown in **FIG. 6A** in disengaged mode and including a connected Bowden cable 64. The Bowden cable 64 (or other flexible conduit or force transmission system) transfers the switch system engagement or disengagement operation from the main body 62 of the switch system 60 to the clutch mechanism 30, in order to activate or deactivate the clutch mechanism 30. The engaged mode of the switch system 60 looks similar to the disengaged mode except the slider 63 would be shifted towards the left in **FIG. 6B** so that it is adjacent to the button cap 64. Any type of switch system may alternatively be employed to activate or deactivate the clutch mechanism 30. The switch system 60 may have more than two states (i.e., more than just 'on' and 'off' states). The switch system 60 may be selected from amongst switch systems of varying lengths or types to accommodate users with different statures or switch system preferences. For example, the length of the Bowden cable may be customized dependent on the user's shape, stature, and/or preference for placement of the main body 62 of the switch system 60 on the user or on the exosuit.

### Modular Clutch Mechanism

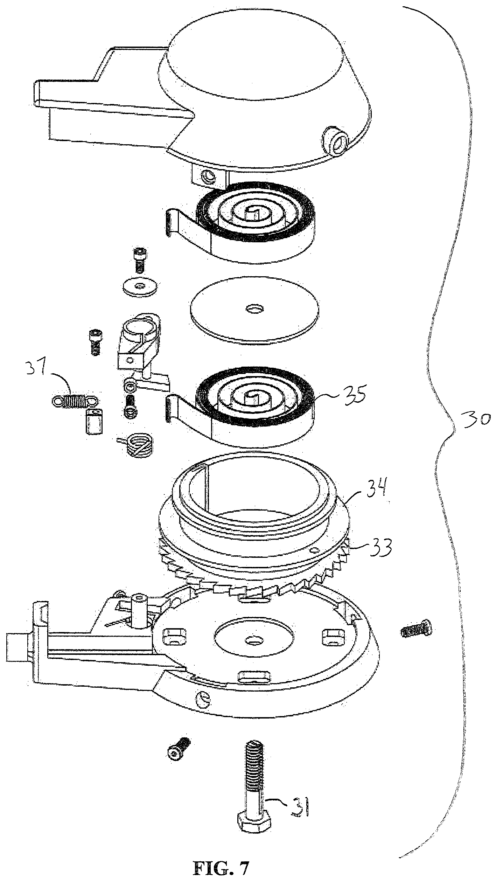

**FIG. 7** is a drawing illustrating an exploded, elevated schematic view of a clutch mechanism 30 for an exosuit. In an embodiment, and as depicted in **FIG. 7****,** a clutch mechanism 30 provides the user of an exosuit with the ability to adjust the force level applied by an elastic member such as elastic band 44a (*see* **FIG. 5**). In an embodiment, the clutch mechanism 30 combines a coil/rotor spring 35 with a rotational pawl and ratchet mechanism 33, and is configured so that the switch system 60 can pull on its Bowden cable 64 that engages (inserts) and disengages (retracts) the pawl into/from the ratchet teeth. The ratchet 33 is directly connected to the spool 34, which is wound with a synthetic rope (or cord, cable, or other elastic component - not shown) and this then connects to the elastic band(s) 44a via connector(s) 44b. The design also includes an extra internal extension spring 37 that ensures that the switch system 60 can be engaged at any time (at any extension length of the coil/rotor spring), and if the pawl is not currently able to slide into a ratchet groove then the extension spring 37 will pull it into place at the next groove just as the spool 34 begins to rotate. In one embodiment, that configuration/design further allows the ratchet 33 to fully reset (recoil) even if the switch system 60 was engaged while someone was leaning forward. In another embodiment, the ratchet 33 is fully locked in position until the clutch is disengaged.

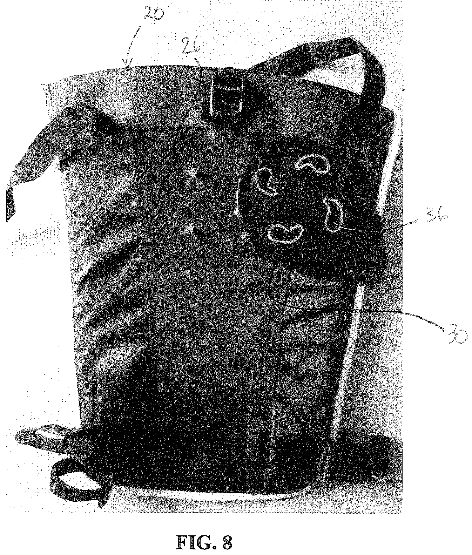

The clutch mechanism 30 may be detachably connected to the back component 20 via a coupler such as bolt 31 (embedded within or attached to the back component 20) or other quick swap mechanism (e.g., hot shoe, magnetic attachment or locking pins/studs (embedded within or attached to the back component 20 - as shown in **FIG. 8****)** with complementary locking holes within the clutch mechanism 30). More particularly, **FIG. 8** is a drawing illustrating a rearward view of a back component 20 and detached clutch mechanism 30 for an exosuit, including locking pins/studs 26 (embedded within or attached to the back component 20) and complementary locking holes 36 on the rear side of the clutch mechanism 30. Upon rotation of the clutch mechanism 30, the locking holes 36 would be arranged to line-up with the locking pins/studs 26, respectively, for a detachable connection. In one embodiment, the clutch mechanism 30 is modular, so that a different clutch mechanism unit/module, style of clutch mechanism, or powered actuator could be quickly swapped in. This would allow for quick and easy maintenance or repairs of the clutch mechanism 30, or alternatively allow for different styles of clutch mechanism 30 to be inserted. For example, there may be applications where the clutch mechanism 30 needs to be made of different materials (e.g., a version for hospitals that does not contain metal in order for it to be used in rooms with certain medical imaging equipment). The clutch mechanism 30 here could be quasi-passive (e.g., with on/off modes) or powered (e.g., motorized to engage and disengage the clutch, or actuated to rotate the spool to change the set point of the elastic band 44a before engaging the clutch). Or the clutch may be replaced with a motor (e.g., electric) or other kind of powered actuator and/or power supply that could be modular and swapped in for other applications (such that those requiring higher or additional levels of assistance). The clutch mechanism 30 may alternatively be located on components other than the back component 20, such as directly on the straps 12 (or anywhere else on the upper-body interface 10) or thigh sleeves 42.

### Additional Modular Components or Accessories

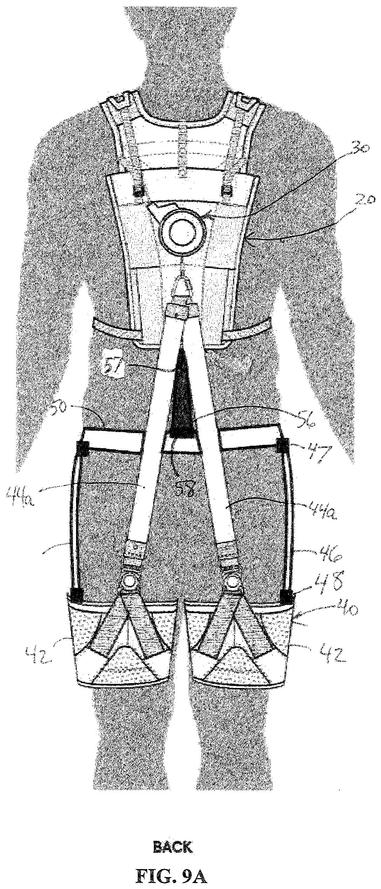

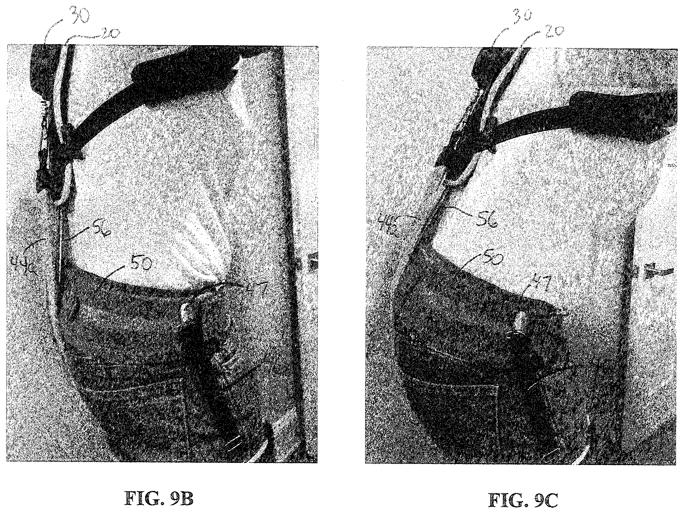

**FIG. 9A** is a drawing illustrating a rearward view of a male on-body rendering of an exosuit with detachable thigh sleeve anchor leads 46 attached to a waist belt 50, and also a detachable (and/or adjustable) linkage 56 between the back component 20 and the waist belt 50. **FIG. 9B** is a drawing illustrating a side view of the exosuit shown in **FIG. 9A****,** depicting the user in a standing position. **FIG. 9C** is a drawing illustrating a side view of the exosuit shown in **FIG. 9A****,** depicting the user in a forward-leaning position.

In one embodiment, there are detachable thigh sleeve anchor leads 46 included in the exosuit. These consist of fabric leads that connect from each thigh sleeve 42 up to the waist. At the waist, the anchor leads 46 connect to an existing belt (that was already worn by the user), or a waist belt 50 that was part of the sleeve anchor system, or to another portion of the users clothing such as the elastic waist materials of their pants (or shorts) or a portion of their shirt. More specifically, the anchor leads 46 detachably connect to the waist belt 50 via connectors 47, and the anchor leads 46 detachably connect to the thigh sleeves 42 via connector 48. The connectors 47, 48 are detachable connectors and may be selected from the types used for connectors 44b, 44c described in this disclosure. A purpose of this embodiment with sleeve anchor leads 46 is to ensure that the thigh sleeves 42 cannot slip or migrate down a users legs, which can happen in certain instances depending on the shape of a person's thighs or the specific pants material they are wearing underneath the thigh sleeves 42, or if the thigh sleeves 42 were not put on tightly enough, or became loosened during use. The thigh sleeve anchor leads 46 can be of adjustable or fixed length. The detachable and modular design of the thigh sleeve anchor leads 46 is useful for personalization and because the thigh sleeve anchor leads 46 are not intended or needed for all users, use cases or embodiments of this exosuit.

In another embodiment, there is a load-bearing linkage 56 between a waist belt 50 and the back component 20, which may be rigid or flexible. More specifically, the linkage 56 detachably or adjustably connects to the back component 20 via connector 57, and the linkage 56 detachably or adjustably connects to the waist belt 50 via connector 58. The connectors 57, 58 are detachable connectors and may be selected from the types used for connectors 44b, 44c described in this disclosure. This linkage 56 may be comprised of a material such as plastic, metal, carbon fiber, or fiber glass. It may be a single piece of material, or may consist of a two or more links or mechanisms which may be connected by rotating or translating joints. This linkage 56 serves to offload a portion of the forces pulling down on the back component 20 directly to the waist or pelvis of the user, which provides enhanced comfort for some users. This is functionally similar to the weight distribution system that is integrated in some fall protection harnesses. However, instead of this component being permanently affixed, the linkage 56 would be modular and swappable since it is not intended or needed for all users, use cases or embodiments of this modular exosuit. The linkage 56 could be adjustable in height to accommodate people of different sizes, or based on the modularity of this system there could be multiple linkage heights available such that the length of linkage was selected to match the height needed for a given individual. Alternatively, the linkage 56 may be permanently affixed at one or more ends, or joints. In one exemplary embodiment, the linkage 56 retracts into the back component 20, for instance using a telescoping assembly, and has a detachable connection to the waist belt 50 and a permanent connection to the back component 20. The connection of the linkage 56 to adjacent components may occur at a fixed point, or alternatively, may be a sliding contact connection. In one exemplary embodiment, the sliding connection allows an elastic linkage, comprised for instance of a carbon fiber beam, to flex and contribute to assistive torque.

Different embodiments could employ linear springs or other types of elastic members. Also, different embodiments could employ other types of clutching mechanisms such as friction-based clutches, cam clutches, overrunning clutches, plate clutches, electromagnetic clutches, positive clutches, buckles, latches, etc. These clutch mechanisms could be passive (unmotorized) or powered (e.g., motorized). Other types of clutch mechanism may alternatively be employed to selectively adjust an assistive force to the user provided by the modular elastic band 44a. These clutching mechanisms include any kind of mode-switching mechanisms that include two or more modes, such as to engage and disengage one or more elastic members, or otherwise adjust the tension in the elastic members. The clutch mechanism may be selectable from amongst multiple clutch mechanisms of varying types or adjustabilities to accommodate different assistive forces to the user provided by the modular elastic band.

Embodiments are directed to a wearable assistance device that includes an upper-body interface, a plurality of fasteners and a back component. The back component is configured to detachably connect to the upper-body interface which is selectable from amongst multiple upper-body interfaces comprising trunk straps of varying shapes or lengths to accommodate users with different shapes or statures. The upper-body interface is configured to be detachably connected to the back component via the plurality of fasteners.

In an embodiment, the trunk straps are male-specific trunk straps and female-specific trunk straps.

In an embodiment, the upper-body interface is in the form of a vest.

In an embodiment, the trunk straps comprise a bottom flap which is configured to be inserted into a pocket within the back component when the upper-body interface is detachably connected to the back component.

Embodiments are also directed to a wearable assistance device that includes an upper-body interface, a lower-body interface, and a modular elastic band. The modular elastic band is detachably connected between the upper-body interface and the lower-body interface.

In an embodiment for this wearable assistance device, the modular elastic band is selectable from amongst multiple modular elastic bands of varying lengths, materials, and/or stiffness properties (e.g., linear, nonlinear, elastic modulus) to accommodate users with different statures, material preferences, or assistance needs.

In an embodiment for this wearable assistance device, the wearable assistance device further comprises a back component connected between the upper-body interface and the lower-body interface. The modular elastic band is detachably connected between the back component and the lower-body interface.

In an embodiment for this wearable assistance device, the upper-body interface is in the form of a vest. The upper-body interface may be standalone, or built into a shirt or other upper-body garment.

In an embodiment for this wearable assistance device, the lower-body interface is in the form of leg sleeves, pants, or shorts. The lower-body interface may be standalone, or built into pants or other lower-body garment.

In an embodiment for this wearable assistance device, the wearable assistance device further comprises a clutch mechanism connected between the back component and the modular elastic band. The clutch mechanism is configured to selectively adjust an assistive force to the user provided by the modular elastic band. In another embodiment, the clutch mechanism is detachably connected to the back component. The clutch mechanism is selectable from amongst multiple clutch mechanisms of varying types or adjustabilities to accommodate different assistive forces to the user provided by the modular elastic band. In a further embodiment, the wearable assistance device further comprises a switch system detachably connected to the clutch mechanism. The switch system may fully detach from the clutch mechanism, or alternatively, the switch system (with its included Bowden cable) and the clutch mechanism (connected to the Bowden cable) may be a sub-system that detaches (and attaches) together as a single module (with multiple points of connection to the exosuit, e.g., the switch also connecting to the straps and the clutch connecting to the back component). Alternatively, the clutch mechanism and switch system are detachable individually and can be replaced independently. The switch system is configured to selectively actuate the clutch mechanism. The switch system is selectable from amongst multiple switch systems of varying lengths or types to accommodate users with different statures or switch system preferences.

Embodiments are further directed to a wearable assistance device that includes an upper-body interface, a lower-body interface, a plurality of fasteners, and a back component. The back component is configured to detachably connect to the upper-body interface which is selectable from amongst multiple upper-body interfaces comprising trunk straps of varying shapes or lengths to accommodate users with different shapes or statures. The upper-body interface is configured to be detachably connected to the back component via the plurality of fasteners.

In an embodiment for this wearable assistance device, the back component is further configured to detachably connect to a selection of multiple elastic bands of varying lengths, materials, or stiffness properties to accommodate users with different statures, material preferences, or assistance needs, and wherein the elastic bands are configured to be detachably connected between the back component and the lower-body interface.

In an embodiment for this wearable assistance device, the trunk straps are male-specific trunk straps and female-specific trunk straps.

In an embodiment for this wearable assistance device, the upper-body interface is in the form of a vest, or other upper-body clothing.

In an embodiment for this wearable assistance device, the lower-body interface is in the form of leg sleeves, pants, or shorts, or other lower-body clothing.

In an embodiment for this wearable assistance device, the wearable assistance device further comprises a clutch mechanism connected to the back component. The clutch mechanism is configured to detachably connect to a selection of multiple elastic bands of varying lengths, materials, or stiffness properties to accommodate users with different statures, material preferences, or assistance needs. The elastic bands are configured to be detachably connected between the clutch mechanism and the lower-body interface. The clutch mechanism is further configured to selectively adjust an assistive force to the user provided by the elastic bands. In another embodiment, the clutch mechanism is detachably connected to the back component. The clutch mechanism is selectable from amongst multiple clutch mechanisms of varying types or adjustabilities to accommodate different assistive forces to the user provided by the elastic bands. In a further embodiment, the wearable assistance device further comprises a switch system detachably connected to the clutch mechanism. The switch system is configured to selectively actuate the clutch mechanism. The switch system is selectable from amongst multiple switch systems of varying lengths or types to accommodate users with different statures or switch system preferences.

### Additional Description

Although embodiments are described above with reference to a back-assist exosuit, wherein the upper-body interface is positioned on the user's torso and the lower-body interface is positioned on the user's thigh(s), the exosuit described in any of the above embodiments may alternatively be an assist exosuit for a different user body part other than the back, wherein the upper-body interface is located on a body part other than the torso, and the lower-body interface is located on a body part other than the thighs. For example, the upper-body interface may be located on a user's bicep (or shoulder) area while the lower-body interface may be located on a user's forearm.

The method steps in any of the embodiments described herein are not restricted to being performed in any particular order. Also, structures or systems mentioned in any of the method embodiments may utilize structures or systems mentioned in any of the device/system embodiments. Such structures or systems may be described in detail with respect to the device/system embodiments only but are applicable to any of the method embodiments.

Features in any of the embodiments described in this disclosure may be employed in combination with features in other embodiments described herein.

## Claims

1. A wearable assistance device comprising:
an upper-body interface (10);
a lower-body interface (40); and
a back component (20) configured to detachably connect to the upper-body interface (10), the upper-body interface (10) comprising trunk straps 12) of varying shapes or lengths to accommodate users with different shapes or statures,
the upper-body interface (10) is configured to be detachably connected to the back component (20) by multiple fasteners (14a/14b/14c/14d/14e);
an elastic member (44a) is connected between the back component (20) and the lower-body interface (40), the elastic member (44a) being detachably connected to the lower-body interface (40) by one or more detachable connectors (44c); **characterized in that**
a clutch mechanism (30) is held by the back component (20) and connected to the elastic member (44a), wherein the clutch mechanism (30) is configured to selectively adjust an assistive force to the user provided by the elastic member (44a).

2. The wearable assistance device of claim 1, wherein the elastic member (44a) is modular and can be selected from amongst multiple modular elastic members of varying lengths, materials, or stiffness properties.

3. The wearable assistance device of claim 2, wherein the elastic member (44a) is detachably connected to the back component (20) via one or more detachable connectors (44b).

4. The wearable assistance device of claim 1, wherein the upper-body interface (10) is in the form of a vest.

5. The wearable assistance device of claim 1, wherein the lower-body interface (40) is in the form of leg sleeves, pants, or shorts.

6. The wearable assistance device of claim 1, wherein the clutch mechanism (30) is detachably connected to the back component via a quick swap mechanism (31).

7. The wearable assistance device of claim 1, wherein the clutch mechanism (30) can be selected from amongst multiple clutch mechanisms of varying types or adjustabilities.

8. The wearable assistance device of claim 1, further comprising a switch system (60) detachably connected to the clutch mechanism (30) wherein the switch system (60) is configured to selectively actuate the clutch mechanism (30).

9. The wearable assistance device of claim 8, wherein the switch system (60) comprises a flexible conduit (64).

10. The wearable assistance device of claim 1, wherein the trunk straps (12) are shaped as male-specific trunk straps or female-specific trunk straps.

11. The wearable assistance device of claim 1, wherein the trunk straps (12) comprise a bottom flap (16) which is configured to be inserted into a pocket (29) of the back component (20) when the upper-body interface (10) is detachably connected to the back component (20).

12. The wearable assistance device of claim 1, wherein the multiple fasteners comprise webbing (14a, 14d) with buckles (14b, 14e).

13. The wearable assistance device of claim 1, wherein the multiple fasteners comprise a hook and loop fastener pad (14c).

14. The wearable assistance device of claim 1, wherein the one or more detachable connectors (44c) comprise a quick-release buckle.

15. The wearable assistance device of claim 4, wherein the back component (20) comprises the clutch mechanism (30).

## Patentansprüche

1. Ein tragbares Hilfsgerät umfassend;
eine Oberkörper-Schnittstelle (20);
eine Unterkörper-Schnittstelle (40); und
eine Rückenkomponente (20), derart konfiguriert, dass sie lösbar mit der Oberkörper-Schnittstelle (10) verbunden werden kann, wobei die Oberkörper-Schnittstelle (10) Rumpfgurte (12) unterschiedlicher Form oder Länge umfasst, um sich an Nutzer mit unterschiedlichen Körperformen oder Statur anzupassen,
die Oberkörper-Schnittstelle (10) derart konfiguriert ist, dass sie über mehrfache Befestigungselemente (14a/14b/14c/14d/14e) lösbar mit der Rückenkomponente (20) verbunden werden kann;
ein elastisches Bauteil (44a) ist zwischen der Rückenkomponente (20) und der Unterkörper-Schnittstelle (40) verbunden, wobei das elastische Bauteil (44a) über ein oder mehrere lösbare Verbinder (44c) lösbar mit der Unterkörper-Schnittstelle (40) verbunden ist; **dadurch gekennzeichnet, dass**
ein Kupplungsmechanismus (30) von der Rückenkomponente (20) gehalten und mit dem elastischen Bauteil (44a) verbunden ist, wobei der Kupplungsmechanismus (30) derart konfiguriert ist, dass er einer dem Nutzer zur Verfügung gestellten Unterstützungskraft des elastischen Bauteils (44a) selektiv anpasst.

2. Die tragbare Hilfsvorrichtung gemäß Anspruch 1, wobei das elastische Bauteil (44a) modular aufgebaut ist und aus mehrfach unterschiedlichen elastischen Bauteilen unterschiedlicher Länge, unterschiedlicher Materialien oder unterschiedlicher Steifigkeitseigenschaften ausgewählt werden kann.

3. Die tragbare Hilfsvorrichtung gemäß Anspruch 2, wobei das elastische Bauteil (44a) mit Hilfe eines oder mehrerer lösbarer Verbinder (44b) lösbar mit der Rückenkomponente (20) verbunden ist.

4. Die tragbare Hilfsvorrichtung gemäß Anspruch 1, wobei die Oberkörper-Schnittstelle (10) die Form einer Weste bildet.

5. Die tragbare Hilfsvorrichtung gemäß Anspruch 1, wobei die Unterkörper-Schnittstelle (40) die Form von Beinhüllen, Hosen oder Shorts bildet.

6. Die tragbare Hilfsvorrichtung gemäß Anspruch 1, wobei der Kupplungsmechanismus (30) mit Hilfe eines Schnellwechselmechanismus (31) lösbar mit der Rückenteil-Komponente verbunden ist.

7. Die tragbare Hilfsvorrichtung gemäß Anspruch 1, wobei der Kupplungsmechanismus (30) aus mehrfach vorhandenen Kupplungsmechanismen unterschiedlicher Art oder Einstellbarkeit ausgewählt werden kann.

8. Die tragbare Hilfsvorrichtung gemäß Anspruch 1, die des Weiteren ein Schaltsystem (60) umfasst, das lösbar mit dem Kupplungsmechanismus (30) verbunden ist, wobei das Schaltersystem (60) derart konfiguriert ist, dass es den Kupplungsmechanismus (30) selektiv betätigt.

9. Die tragbare Hilfsvorrichtung gemäß Anspruch 8, wobei das Schaltsystem (60) eine flexible Leitung (64) umfasst.

10. Die tragbare Hilfsvorrichtung gemäß Anspruch 1, wobei die Rumpfgurte (12) als männerspezifische Rumpfgurte oder frauenspezifische Rumpfgurte ausgebildet sind.

11. Die tragbare Hilfsvorrichtung gemäß Anspruch 1, wobei die Rumpfgurte (12) eine untere Lasche (16) umfasst, derart konfiguriert, dass sie in eine Tasche (29) der Rückenkomponente (20) eingeführt wird, wenn die Oberkörper-Schnittstelle (10) lösbar mit der Rückenkomponente (20) verbunden ist.

12. Die tragbare Hilfsvorrichtung gemäß Anspruch 1, wobei die mehrfachen Befestigungselemente Gurtbänder (14a, 14d) mit Schnallen (14b, 14e) umfassen.

13. Die tragbare Hilfsvorrichtung gemäß Anspruch 1, wobei die mehrfachen Befestigungselemente ein Klettverschlusspolster (14c) umfassen.

14. Die tragbare Hilfsvorrichtung gemäß Anspruch 1, wobei die ein oder mehrere lösbaren Verbinder (44c) eine Schnellverschlussschnalle umfassen.

15. Die tragbare Hilfsvorrichtung gemäß Anspruch 4, wobei die Rückenkomponente (20) den Kupplungsmechanismus (30) umfasst.

## Revendications

1. Dispositif d'assistance portable comprenant :
une interface pour le haut du corps (10) ;
une interface pour le bas du corps (40) ; et
un élément dorsal (20) conçu pour se fixer de manière amovible à l'interface pour le haut du corps (10), l'interface pour le haut du corps (10) comprenant des sangles de tronc (12) de formes ou de longueurs variables afin de s'adapter à des utilisateurs présentant des morphologies ou des statures différentes,
l'interface pour le haut du corps (10) étant conçue pour être reliée de manière amovible au composant dorsal (20) par plusieurs éléments de fixation (14a/14b/14c/14d/14e) ;
un élément élastique (44a) est relié entre le composant dorsal (20) et l'interface du bas du corps (40), l'élément élastique (44a) étant relié de manière amovible à l'interface du bas du corps (40) par un ou plusieurs connecteurs amovibles (44c) ;
**caractérisé en ce que**
un mécanisme d'embrayage (30) est maintenu par le composant dorsal (20) et relié à l'élément élastique (44a), le mécanisme d'embrayage (30) étant configuré pour ajuster de manière sélective la force d'assistance fournie à l'utilisateur par l'élément élastique (44a).

2. Dispositif d'assistance portable selon la revendication 1, dans lequel l'élément élastique (44a) est modulaire et peut être choisi parmi plusieurs éléments élastiques modulaires de longueurs, de matériaux ou de propriétés de rigidité variables.

3. Dispositif d'assistance portable selon la revendication 2, dans lequel l'élément élastique (44a) est relié de manière amovible au composant dorsal (20) via un ou plusieurs connecteurs amovibles (44b).

4. Dispositif d'assistance portable selon la revendication 1, dans lequel l'interface du haut du corps (10) se présente sous la forme d'un gilet.

5. Dispositif d'assistance portable selon la revendication 1, dans lequel l'interface du bas du corps (40) se présente sous la forme de manchons pour les jambes, d'un pantalon ou d'un short.

6. Dispositif d'assistance portable selon la revendication 1, dans lequel le mécanisme d'embrayage (30) est relié de manière amovible au composant dorsal par l'intermédiaire d'un mécanisme de remplacement rapide (31).

7. Dispositif d'assistance portable selon la revendication 1, dans lequel le mécanisme d'embrayage (30) peut être choisi parmi plusieurs mécanismes d'embrayage de types ou de niveaux de réglage variés.

8. Dispositif d'assistance portable selon la revendication 1, comprenant en outre un système de commutation (60) relié de manière amovible au mécanisme d'embrayage (30), dans lequel le système de commutation (60) est configuré pour actionner de manière sélective le mécanisme d'embrayage (30).

9. Dispositif d'assistance portable selon la revendication 8, dans lequel le système de commutation (60) comprend un conduit flexible (64).

10. Dispositif d'assistance portable selon la revendication 1, dans lequel les sangles de torse (12) sont conçues comme des sangles de torse spécifiques aux hommes ou comme des sangles de torse spécifiques aux femmes.

11. Dispositif d'assistance portable selon la revendication 1, dans lequel les sangles de torse (12) comprennent un rabat inférieur (16) qui est configuré pour être inséré dans une poche (29) du composant dorsal (20) lorsque l'interface du haut du corps (10) est reliée de manière amovible au composant dorsal (20).

12. Dispositif d'assistance portable selon la revendication 1, dans lequel les multiples éléments de fixation comprennent des sangles (14a, 14d) munies de boucles (14b, 14e).

13. Dispositif d'assistance portable selon la revendication 1, dans lequel les multiples éléments de fixation comprennent un patin à fermeture auto-agrippante (14c).

14. Dispositif d'assistance portable selon la revendication 1, dans lequel le ou les connecteurs amovibles (44c) comprennent une boucle à dégagement rapide.

15. Dispositif d'assistance portable selon la revendication 4, dans lequel le composant dorsal (20) comprend le mécanisme d'embrayage (30).
